# EUROPEAN PATENT SPECIFICATION

(11) **EP 4 376 936 B1**
(45) Date of publication and mention of the grant of the patent: **10.06.2026**
(21) Application number: 22772592.6
(22) Date of filing: 30.08.2022
(51) Int. Cl.: A61N 1/04, A61N 1/06, A61N 1/40, A61B 5/268

(54) **ELECTRODE ASSEMBLY COMPRISING AN ANISOTROPIC MATERIAL LAYER AND A SKIN CONTACT LAYER COMPRISING A CONDUCTIVE ADHESIVE COMPOSITE**
ELEKTRODENANORDNUNG MIT EINER ANISOTROPEN MATERIALSCHICHT UND EINER HAUTKONTAKTSCHICHT MIT EINEM LEITFÄHIGEN HAFTVERBUNDSTOFF
ENSEMBLE ÉLECTRODE COMPRENANT UNE COUCHE DE MATÉRIAU ANISOTROPE ET UNE COUCHE DE CONTACT AVEC LA PEAU COMPRENANT UN COMPOSITE ADHÉSIF CONDUCTEUR

(30) Priority: 31.08.2021 US 202163239173 P; 30.06.2022 US 202263357441 P
(43) Date of publication of application: 05.06.2024
(73) Proprietor: Novocure GmbH, 6340 Baar (CH)
(72) Inventor: SHNAIDERMAN, Rosa, 3190500 Haifa (IL); WASSERMAN, Yoram, 3190500 Haifa (IL); OBUCHOVSKY, Stas, 3190500 Haifa (IL); KUPLENNIK, Nataliya, 3190500 Haifa (IL); SHAPIRO, David, 3190500 Haifa (IL)
(74) Representative: Carpmaels & Ransford LLP
(86) International application number: PCT/IB2022/058124
(87) International publication number: WO 2023/031792

(56) References cited:
- US-A1- 2005 015 134
- US-A1- 2010 185 078
- US-A1- 2013 066 412
- US-A1- 2020 171 297

## Description

### BACKGROUND

Tumor Treating Fields (TTFields) therapy is a proven approach for treating tumors using alternating electric fields at frequencies between 50 kHz - 1 MHz, more commonly, 100-500 kHz. In current commercial systems, the alternating electric fields are induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

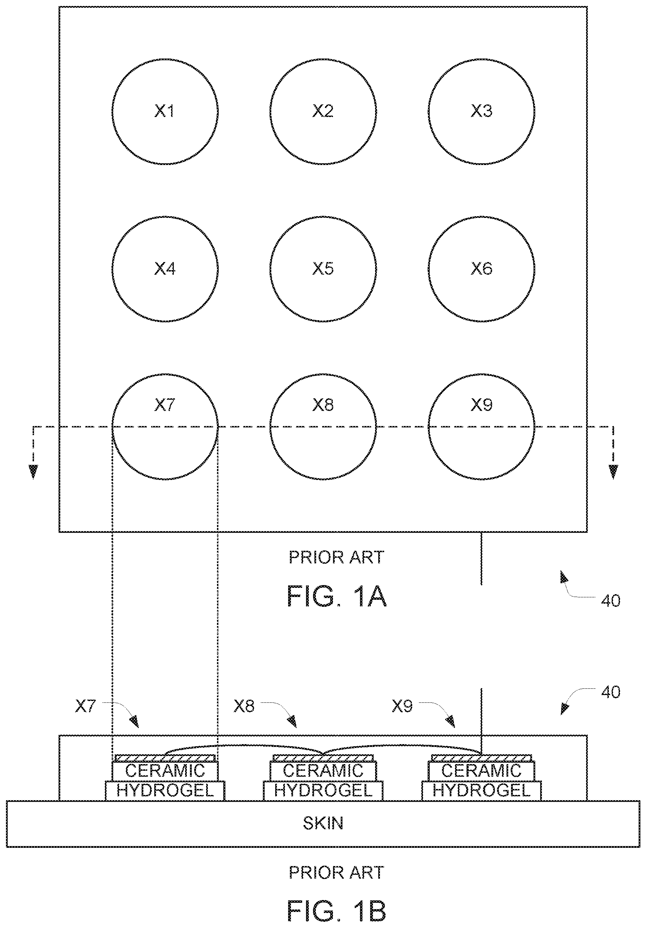

FIG. 1A is a schematic representation of a prior art electrode assembly 40 including nine prior art electrode elements, labeled X1-X9. FIG. 1B is a cross sectional schematic view of electrode elements X7-X9 of the electrode assembly 40, taken along the dashed line in FIG. 1A.

As shown in FIG. 1B, electrode element X7 (taken as exemplary) includes a metal layer (shown with diagonal hatching) and a ceramic (dielectric) layer. A respective layer of electrically conductive hydrogel is provided between each ceramic layer and the subject's skin, to ensure good electrical contact of the electrode elements with the body. An AC voltage from an AC voltage generator (not shown) is applied to the metal layers of electrode elements in opposing electrode assemblies to generate the TTFields in the subject's body. In order to retain the electrode assembly in place during use, an adhesive cover (bandage) is typically provided over the electrode assembly.

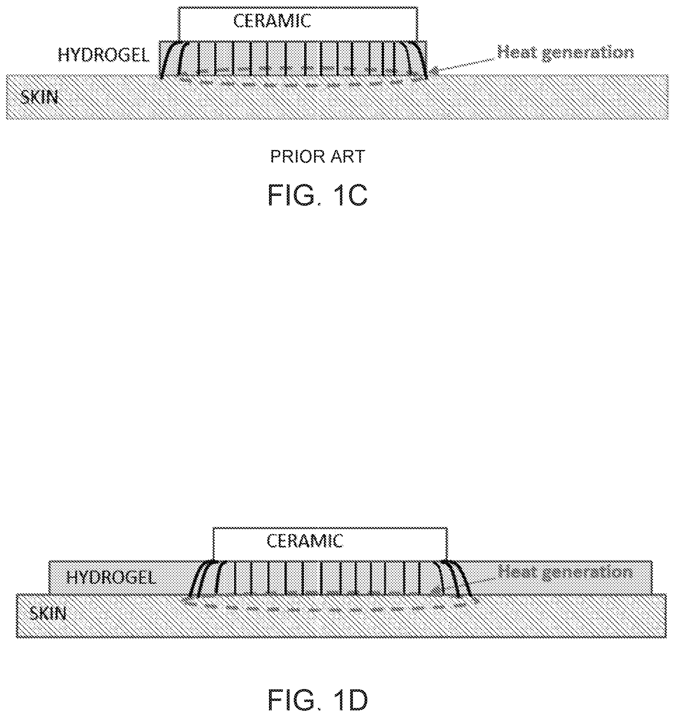

During use, the hydrogel and the skin under the electrode elements heat up, and safety considerations require that the skin temperature remain below a safety threshold (e.g., 41° C). Because the vast majority of the heat appears immediately below the electrode elements X1-X9 (as shown in FIG. 1C), the prior art electrode assembly has hot spots immediately below the electrode elements, and cooler regions positioned between the electrode elements. And those hot spots limit the amount of current that can be delivered through the prior art electrode assemblies.

The hydrogel layer(s) of the electrode assembly can also present various issues. For example, since the hydrogel has a limited shelf-life, moisture barrier packaging is required, increasing the cost of packaging for the electrode assembly. Additionally, the signal through the hydrogel can vary with the specific moisture content within the hydrogel, and the hydrogel can fail with either too much or too little water. Further, during use, electrode assemblies having hydrogel layers must be changed out frequently, and many patients have adverse reactions (e.g., allergic reactions) to the hydrogel. And, as a skin-contact layer, the hydrogel layer tends to transfer to the subject's skin if the array is released or repositioned. A skin contact structure that provides sufficient conductivity through its thickness, and avoids the issues of using hydrogel, is desirable.

US-A-2013/0066412 discloses an electrode assembly that includes an electrode configured to provide electrical contact with a subject's skin through a subject contact surface. The electrode includes an electrode base pad having a first contact surface on a side of the electrode opposite the subject contact surface, a connector assembly configured to electrically connect the electrode to an external electrical apparatus to transfer electrical signals between the subject's skin and the external electrical apparatus and including a housing having a second contact surface configured to be magnetically and electrically coupled to the first contact surface.

US-A-2010/0185078 discloses a bioelectrode comprising a skin-side, electrically-conducting adhesive layer and a flexible, electrical-connecting cable which, in an electrically-insulating cable sheath, includes at least one electrical conductor, and fitted at the electrode-side end of the connecting cable is a preassembled electrical-conducting element electrically connected to the electrical conductor of the connecting cable, where the preassembled electrical-conducting element is electrically connected in the installed condition to the adhesive layer.

US-A-2005/0015134 discloses a biomedical electrode that distributes current over the entire surface of a conductive polymeric sheet using a current-spreading layer located on the upper surface of the conductive polymeric sheet (i.e., the surface facing away from the patient). The conductive polymeric sheet includes a conductive undercoating on its lower surface (i.e., the surface facing the patient), and an electrolyte layer (e.g., a hydrogel pressure-sensitive adhesive) is located on the bottom of the biomedical electrode, with the conductive undercoating being located between the electrolyte layer and the conductive polymeric sheet.

### SUMMARY

One aspect of the invention is directed to an apparatus for applying an alternating electric field to a subject's body according to claim 1.

Embodiments of the invention are disclosed in the dependent claims.

### BRIEF DESCRIPTION OF THE DRAWINGS

FIG. 1A is a schematic representation of a prior art electrode assembly.
FIG. 1B is a cross sectional view of electrode elements of the prior art electrode assembly, taken along the dashed line in FIG. 1A.
FIG. 1C is a cross sectional view showing the heat generation properties of a prior art electrode element.
FIG. 1D is a cross sectional view showing the heat generation properties of a hypothetical modification to the FIG. 1B electrode element.
FIG. 2 is a plan schematic representation of an electrode assembly including electrode elements that is used for applying TTFields to a subject's body.
FIG. 3A is a cross sectional representation of a first embodiment including electrode elements E1, E2, taken along the dashed line in FIG. 2.
FIG. 3B is a cross sectional view showing the heat generation properties of the FIG. 3A embodiment.
FIG. 4A is a thermal image of a prior art electrode assembly.
FIG. 4B is a thermal image of an electrode assembly corresponding to the FIG. 3A embodiment.
FIG. 4C is a graph comparing the thermal properties of the prior art electrode assembly with the FIG. 3A embodiment.
FIG. 5 is a cross sectional representation of a second embodiment including electrode elements E1, E2, taken along the dashed line in FIG. 2.
FIG. 6 is a cross sectional representation of a third embodiment that includes a single electrode element E1.
FIG. 7 is a cross sectional representation of a fourth embodiment that includes a single electrode element E1.
FIG. 8 is a cross sectional representation of a fifth embodiment that includes a single electrode element E1.
FIG. 9 is a block diagram of a system incorporating two electrode assemblies that is used for applying TTFields to a subject's body.
FIGS. 10A-10B show a modified version of the embodiment of FIGS. 3A-3B. As shown, FIGS. 10A-10B include a skin contact layer comprising a conductive adhesive composite rather than hydrogel.
FIG. 11 shows a modified version of the embodiment of FIG. 5. As shown, FIG. 11 includes a skin contact layer comprising a conductive adhesive composite rather than hydrogel.
FIG. 12 shows a modified version of the embodiment of FIG. 6. As shown, FIG. 12 includes a skin contact layer comprising a conductive adhesive composite rather than hydrogel.
FIG. 13 shows a modified version of the embodiment of FIG. 7. As shown, FIG. 13 includes a skin contact layer comprising a conductive adhesive composite rather than hydrogel.
FIG. 14 shows a modified version of the embodiment of FIG. 8. As shown, FIG. 14 includes a skin contact layer comprising a conductive adhesive composite rather than hydrogel.
FIG. 15 shows a modified version of the embodiment of FIG. 13. As shown, FIG. 15 does not include a layer of anisotropic material.
FIG. 16 shows a modified version of the block diagram of FIG. 9. As shown, the electrode assemblies within the block diagram of FIG. 16 include a conductive adhesive composite and do not require a layer of anisotropic material.
FIG. 17 shows a modified version of the embodiment of FIG.10A. As shown, the embodiment has a skin contact structure in place of the front layer of conductive adhesive composite.
FIG. 18 shows a modified version of the embodiment of FIG. 11, having a skin contact structure in place of the front layer of conductive adhesive composite.
FIG. 19 shows a modified version of the embodiment of FIG. 12, having a skin contact structure in place of the front layer of conductive adhesive composite.
FIG. 20 shows a modified version of the embodiment of FIG. 13, having a skin contact structure in place of the front layer of conductive adhesive composite.
FIG. 21 shows a modified version of the embodiment of FIG. 14, having a skin contact structure in place of the front layer of conductive adhesive composite.

Various embodiments are described in detail below with reference to the accompanying drawings, wherein like reference numerals represent like elements, and wherein descriptions of like elements may not be repeated for every embodiment, but may be considered to be the same if previously described herein.

### DESCRIPTION OF THE PREFERRED EMBODIMENTS

This application describes exemplary electrode assemblies that may be used, e.g., for delivering TTFields to a subject's body and treating one or more cancers or tumors located in the subject's body.

When TTFields are applied to a subject's body, the temperature at the subject's body may increase proportionally to the induced electric field. Regulations limit the amount of current that can be driven through a transducer array to an amount that keeps the measured temperature at locations on the subject's body below a temperature threshold. As practiced in the art, the temperature at the location of the transducer arrays on the subject's body is controlled to be below the temperature threshold by reducing the operational current driven by the transducer arrays and reducing the strength of the resulting TTFields. This in turn becomes an over-riding limitation on the TTFields strength that can be used to treat the tumor. Accordingly, there is a need in the art to safely access higher TTField strengths without exceeding the temperature threshold at the subject's skin.

On transducer arrays that comprise multiple electrode elements, the portions of the transducer arrays positioned directly beneath the electrode elements get hotter than the portions of the transducer arrays positioned between the electrode elements. Furthermore, on transducer arrays that comprise multiple electrode elements, higher currents flow through the electrode elements located along the edge of the array compared to the electrode elements located toward the middle of the array. Further still, an electrode element located at a corner or similar sharp bend in the edge of the array will have a higher current than other electrode elements along the edge and near the center of the array. The tendency of a transducer array to drive higher currents through electrode elements located along the edge of the array, and particularly at the corners, is referred to herein as the "edge effect."

An uneven distribution of current through the transducer array due to either the distribution of the electrode elements or the edge effect can lead to higher temperature zones (or "hot spots") e.g., at the corners or edges of the transducer array. These hot spots are the locations that reach the threshold temperature first and therefore control the requirement to reduce the current. As such, the generation of hot spots limits the maximum operational current that may be driven by a transducer array, and the strength of the resulting TTFields.

The inventors have now recognized that a need exists for transducer arrays that reduce or minimize uneven distribution of current and thereby allow the application of higher operating currents. Transducer arrays operated with increased current can induce stronger TTFields in the subject's body, ultimately leading to better patient outcomes. A related issue with respect to hot-spots under specific electrodes is an increased risk of skin irritation at those locations. The sensitivity to skin irritation is accentuated by the hydrogel skin contact layer, which is acidic and has a high ion content. Accordingly, avoiding hot spots and replacing the hydrogel with a less skin-irritating conductive gel or conductive adhesive would be desirable. The electrode assemblies disclosed herein allow current and heat to be spread evenly over the array thereby minimizing or eliminating hot spots; and the use of conductive adhesives, in some embodiments described herein, can reduce skin irritation without adhesive transfer to the subject's skin upon removal or repositioning of the arrays.

Optionally, the embodiments described herein incorporate into the electrode assembly a sheet of material having anisotropic thermal properties and/or anisotropic electrical properties, as described below. If the sheet of material has anisotropic thermal properties, then the sheet spreads the heat out more evenly over a larger surface area. If the sheet of material has anisotropic electrical properties, then the sheet spreads the current out more evenly over a larger surface area. In each case, this lowers the temperature of the hot spots and raises the temperature of the cooler regions when a given AC voltage is applied to the electrode assembly (as compared to the prior art configuration described above). Accordingly, the current can be increased (thereby increasing the therapeutic effect) without exceeding the safety temperature threshold at any point on the subject's skin.

In some embodiments, the anisotropic material is anisotropic with respect to electrical conductivity properties. In some embodiments, the anisotropic material is anisotropic with respect to thermal conductivity properties. In some preferred embodiments, the anisotropic material is anisotropic with respect to both electrical conductivity properties and thermal conductivity properties.

The anisotropic thermal properties include directional thermal properties. Specifically, the sheet has a first thermal conductivity in a direction that is perpendicular to its front face (skin-facing surface). And the thermal conductivity of the sheet in directions parallel to the front face is more than two times higher than the first thermal conductivity. In some preferred embodiments, the thermal conductivity in the parallel directions is more than ten times higher than the first thermal conductivity. For example, the thermal conductivity of the sheet in directions that are parallel to the front face may be more than: 1.5 times, 2 times, 3 times, 5 times, 10 times, 20 times, 100 times, 200 times, or even more than 1,000 times higher than the first thermal conductivity.

The anisotropic electrical properties include directional electrical properties. Specifically, the sheet has a first resistance in a direction that is perpendicular to its front face. And resistance of the sheet in directions parallel to the front face is less than the first resistance. In some preferred embodiments, the resistance in the parallel directions is less than half of the first resistance or less than 10% of the first resistance. For example, the resistance of the sheet 70 in directions that are parallel to the front face may be less than: 75%, 50%, 40%, 30%, 20%, 10%, 5%, 1%, 0.5%, or even less than 0.1% of the first resistance.

In some embodiments (e.g., when the sheet of anisotropic material is a sheet of pyrolytic graphite), the sheet of anisotropic material has both anisotropic electrical properties and anisotropic thermal properties.

In some embodiments (e.g., when the sheet of anisotropic material is a sheet of pyrolytic graphite), the sheet of anisotropic material is nonmetallic. These embodiments are particularly advantageous in situations where preventing the transfer of ions into a subject's body is desirable. More specifically, using a metallic sheet could result in the transfer of ions into a subject's body. In situations where this is not desirable, embodiments that use nonmetallic sheets of anisotropic material are preferable.

The present invention can be understood more readily by reference to the following detailed description, examples, drawings, and claims, and their previous and following description. However, it is to be understood that this invention is not limited to the specific apparatus described hereinafter, but the scope of the invention is defined by the appended claims.

As used in the specification and the appended claims, the singular forms "a," "an" and "the" include plural referents unless the context clearly dictates otherwise.

In the preceding and following description, the terms "front" and "skin-facing" are used interchangeably to refer to a face or surface of the disclosed electrode assemblies (or components thereof) that faces or is oriented toward the skin of a subject (or generally toward the body of a subject) when used as disclosed herein. Similarly, the terms "rear" and "outwardly facing" are used interchangeably to refer to a face or surface of the disclosed electrode assemblies (or components thereof) that faces away from or is oriented away from the skin of a subject (or generally away from the body of the subject) when used as disclosed herein.

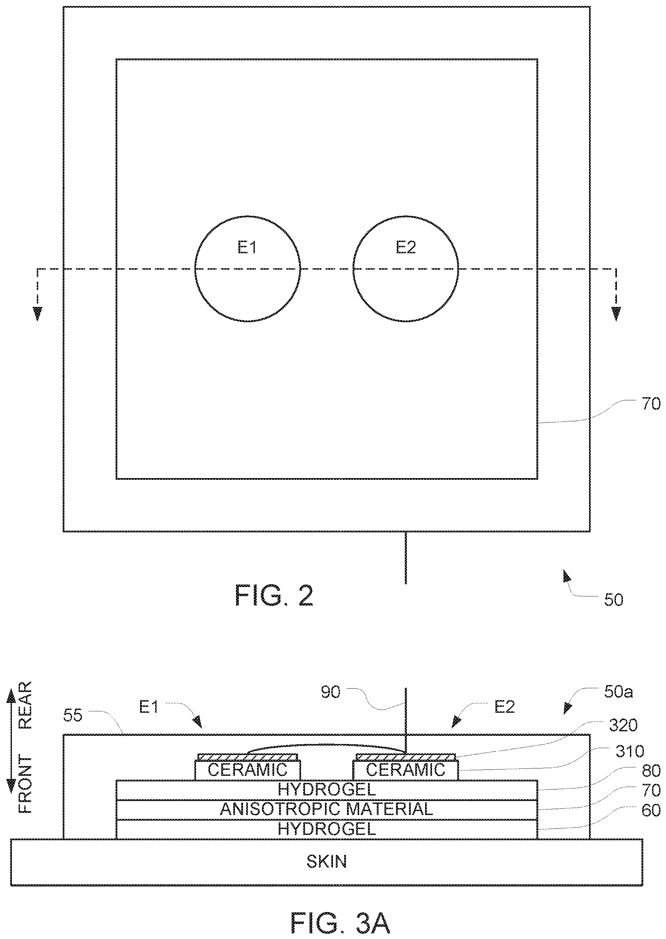

FIG. 2 is a schematic representation of an electrode assembly 50 of an embodiment including electrode elements used for applying TTFields to a subject's body. In FIG. 2, only two electrode elements labeled E1 and E2 are shown, but additional electrode elements may be included in the electrode assembly 50. In alternative embodiments, the electrode assembly 50 includes only a single electrode element. Notably, FIG. 2 depicts an electrode assembly 50 generically, and those electrode assemblies E1 and E2 can have different configurations (e.g., as described below in connection with FIGS. 3A-8, 10A-15, and 17-21).

FIG. 3A is a cross sectional representation of a first embodiment of an electrode assembly 50a including electrode elements E1, E2, taken along the dashed line in FIG. 2.

In the FIG. 3A embodiment, the electrode assembly 50a includes a sheet of anisotropic material 70 having a front face (facing towards the subject's skin in FIG. 3A) and a rear face (outwardly facing). This sheet 70 has a first thermal conductivity in a direction that is perpendicular to the front face. Thermal conductivity of the sheet 70 in directions that are parallel to the front face is more than two times higher than the first thermal conductivity. In some preferred embodiments, the thermal conductivity of the sheet 70 in directions that are parallel to the front face is more than ten times higher than the first thermal conductivity. The sheet 70 in the FIG. 3A embodiment is also anisotropic in another respect. More specifically, the sheet 70 has a first resistance in a direction that is perpendicular to the front face, and the resistance of the sheet in directions that are parallel to the front face is less than half of the first resistance. In some embodiments, the resistance of the sheet in directions that are parallel to the front face is less than 10% of the first resistance.

The electrode assembly 50a includes a sheet of conductive anisotropic material 70 having a front face (facing towards the subject's skin in FIG. 3A) and a rear face. The sheet of conductive anisotropic material 70 could be a sheet of graphite. Examples of suitable forms of graphite include synthetic graphite, such as pyrolytic graphite (including, but not limited to, Pyrolytic Graphite Sheet (PGS), available from Panasonic Industry, Kadoma, Osaka, Japan), other forms of synthetic graphite, including but not limited to, graphite foil made from compressed high purity exfoliated mineral graphite (including, but not limited to, that supplied by MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan. In alternative embodiments, conductive anisotropic materials other than graphite may be used instead of graphite.

In some embodiments, the sheet of anisotropic material 70 is a sheet of pyrolytic graphite. Thermal conductivity of pyrolytic graphite sheets in directions that are parallel to the front face of those sheets (i.e., in the a-b plane) is typically more than 50 times higher than the thermal conductivity of those sheets in directions that are perpendicular to the front face (i.e., in the c direction). And electrical resistivity of pyrolytic graphite sheets in directions that are parallel to the front face of those sheets (i.e., in the a-b plane) is typically less than 2% of the electrical resistivity of those sheets in directions that are perpendicular to the front face (i.e., in the c direction).

In other embodiments, the sheet of anisotropic material 70 is graphite foil made from compressed high purity exfoliated mineral graphite or graphitized polymer film. In other embodiments, the anisotropic material may be pyrolytic carbon. Other embodiments may utilize sheets of other conducting materials with anisotropic properties. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

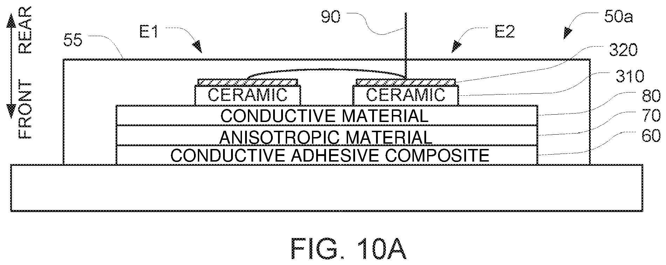

The electrode assembly 50a further includes at least one layer of conductive material 60 disposed on the front face of the sheet 70, and the at least one layer of conductive material 60 has a biocompatible front surface. Note that in the embodiment illustrated in FIG. 3A, there is only a single layer of conductive material 60, and that single layer (the front layer) is biocompatible. But in alternative embodiments (described herein) there could be more than one layer, in which case only the front layer must be biocompatible. In the FIG. 3A embodiment, the front layer of material 60 is configured to ensure good electrical contact between the device and the body. In some embodiments, the front layer of material 60 should cover the entire front face of the sheet of anisotropic material 70. The front layer of material 60 may be the same size or larger than the sheet of anisotropic material 70. In some embodiments, and as shown in FIG. 3A, the front layer of conductive material 60 comprises hydrogel. In these embodiments, the hydrogel may have a thickness between 50 and 2000 µm. In other embodiments, and as shown in FIG. 10A, the front layer of conductive material 60 comprises a conductive adhesive composite as further disclosed herein.

In some embodiments, the at least one layer of conductive material 60 is a single layer of non-hydrogel biocompatible conductive adhesive such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon; or ARcare^{®} 8006 electrically conductive adhesive composition manufactured and sold by Adhesives Research, Inc. (Glen Rock, PA, USA). Non-hydrogel conductive adhesives may comprise a waterless polymer with adhesive properties and carbon particles, powder, fibers, flakes or nanotubes. The adhesive polymer may be, for example, an acrylic polymer or a silicone polymer, or combination thereof, which may be available as acrylic- or silicone-based carbon-filled adhesive tapes. The adhesive may additionally include one or more conductive polymer (such as, for example, polyaniline (PANI), or poly(3,4-ethylenedioxythiophene (PEDOT), or others known in the art). The conductive filler in the at least one layer of conductive material 60 can be non-metallic. In these embodiments, the biocompatible conductive adhesive may have a thickness between 10 and 2,000 µm, such as, from 20 to 1000 µm, or 30 to 400 µm.

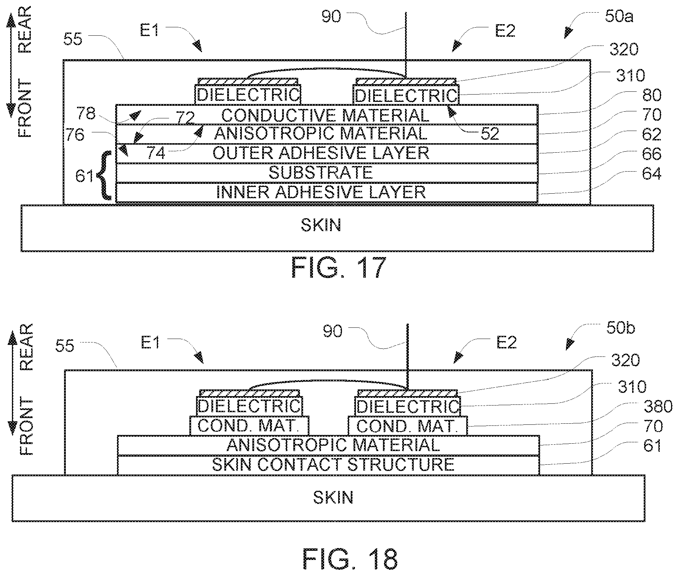

In the FIG. 3A embodiment, the electrode assembly 50a further includes a first electrode element E1 positioned behind the sheet 70. The first electrode element E1 has a first front (skin-facing) face disposed in electrical contact with the rear (outwardly facing) face of the sheet 70. In the FIG. 3A embodiment, the first electrode element E1 includes a first layer of dielectric (e.g., ceramic) material 310 having a front (skin-facing) face and a rear (outwardly facing) face, and a first layer of metal 320 disposed on the rear face of the first layer of dielectric material 310. The front face of the first layer of dielectric material 310 is the first front face of the first electrode element E1. As further illustrated in FIGS. 17-18, the dielectric material need not be ceramic. In some aspects, for example, the dielectric material 310 can comprise polymer (e.g., high dielectric constant polymer). Accordingly, it should be understood that, in all embodiments disclosed herein, the dielectric material 310 referred to and shown in the drawings as ceramic can be any suitable dielectric material (for example, a polymer layer having a dielectric constant of at least 10, or another material having a dielectric constant of at least 10).

In some embodiments, the layer of dielectric material 310 can have a dielectric constant ranging from 10 to 50,000. In some embodiments, the layer of dielectric material 310 comprises a high dielectric polymer material such as poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylene-chlorotrifluorocthylcne-chlorofluoroethylene) or "Poly(VDF-TrFE-CTFE-CFE)."

In some embodiments, the layer of dielectric material 310 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In some embodiments, the sheet 70 has a centroid, and the centroid of the first front face of the first electrode element E1 is positioned less than 3 cm away from the centroid of the sheet 70. In some embodiments, the sheet 70 has a centroid and a dimension parallel to the rear face of the sheet 70 (e.g., a length or a width), and the centroid of the first front face of the first electrode element E1 is positioned away from the centroid of the sheet 70 by less than 10% of the dimension.

The electrode assembly 50a further includes a first layer of conductive material 80 positioned between the first front face of the first electrode element E1 (i.e., the front face of the first layer of dielectric material 310) and the rear face of the sheet 70. The first layer of conductive material 80 facilitates the electrical contact between the first front face of the first electrode element E1 and the rear face of the sheet 70. In the illustrated embodiment, the layer of conductive material 80 is a layer of hydrogel. But in alternative embodiments, for example, as shown in FIG. 10A, a different conductive material (e.g., conductive grease, conductive adhesives, conductive tape, etc.) could be used. For example, in FIG. 10A, the layer of conductive material 80 can comprise a conductive adhesive composite as further disclosed herein.

The electrode assembly 50a may optionally include one or more additional electrode elements. In the illustrated embodiment, the electrode assembly 50a includes a second electrode element E2 positioned behind the sheet 70. The second electrode element E2 has a second front (skin-facing) face disposed in electrical contact with the rear face of the sheet 70. The two electrode elements E1, E2 in FIG. 3A have identical structures. Thus, the second electrode element E2 includes a second layer of dielectric (e.g., ceramic) material 310 having a front (skin-facing) face and a rear (outwardly facing) face, and a second layer of metal 320 disposed on the rear face of the second layer of dielectric material 310. The front face of the second layer of dielectric material 310 is the second front face of the second electrode element E2. In some embodiments, the collective area of all the electrode elements is less than the area of the sheet 70, less than half the area of the sheet 70, less than one quarter the area of the sheet 70, or less than one tenth the area of the sheet 70.

The first layer of conductive material 80 is positioned between the second front face of the second electrode element E2 (i.e., the front face of the second layer of dielectric material 3 10) and the rear face of the sheet 70. The first layer of conductive material 80 facilitates the electrical contact between the second front face of the second electrode element E2 and the rear face of the sheet 70. As described for E1, and as shown in FIG. 3A, the conductive material 80 may be a layer of hydrogel, but in alternative embodiments, a different conductive material may be used (e.g., conductive grease, conductive adhesives, conductive tape, etc.). For example, in FIG. 10A, the layer of conductive material 80 can comprise a conductive adhesive composite as further disclosed herein.

The metal layers 320 of all of the electrode elements (i.e., E1 and E2 in the illustrated embodiment), may be wired together (e.g., using wires, traces on a flex circuit, etc.) to a lead 90. The lead 90 supplies an AC voltage from an AC voltage generator (not shown) to the electrode elements to generate the TTFields when the electrode assembly 50a is affixed to the subject's body for treatment.

Optionally, the electrode assembly 50a includes a flexible self-adhesive backing 55 configured to support the sheet 70, the first electrode element E1 (and any other electrode elements present in the electrode assembly), and the front layer of conductive material 60 so that the front layer of conductive material 60 can be positioned against the subject's skin.

As noted above, FIG. 2 is a plan schematic representation of an electrode assembly 50 including electrode elements E1, E2. This view of FIG. 2 (not to scale) also demonstrates that the area of the sheet 70 is larger (e.g., at least 2 times larger, at least 4 times larger, or at least 10 times larger) than the combined areas of the electrode elements E1, E2. When an AC voltage is applied to the electrode elements E1, E2, heat spreads out across the entire sheet 70, which minimizes or eliminates hot spots. In addition to spreading and altering the directionality of the heat (minimizing heat in specific locations), the spread has an additional effect in dissipating the heat since the majority is directed in the x-y plane to the edges of the sheet, which terminate in a room temperature (cooler) heat sink, rather than proceeding in the z-direction to a much warmer body temperature. The area therefore cools much faster.

This reduction in hot spots (as compared to the prior art) becomes apparent by comparing FIG. 1C to FIG. 3B. More specifically, FIG. 1C shows the current distribution and heat generation for prior art electrode elements, each of which is positioned on a conductive hydrogel layer that is about the same size as the electrode element. As shown in FIG. 1C, all the current passes through the hydrogel layer directly beneath the electrode elements, which results in hot spots directly beneath the electrode elements.

One might initially think that this problem could be solved by increasing the area of the hydrogel to cover all the regions between the electrode elements (i.e., by covering a significantly larger area in the x-y plane than that of the electrode elements). But this is not the case. More specifically, FIG. 1D shows the current distribution and heat generation for this hypothetical electrode assembly. As shown in FIG. 1D, all the current still passes through the hydrogel layer directly beneath the electrode elements, which results in hot spots directly beneath the electrode elements.

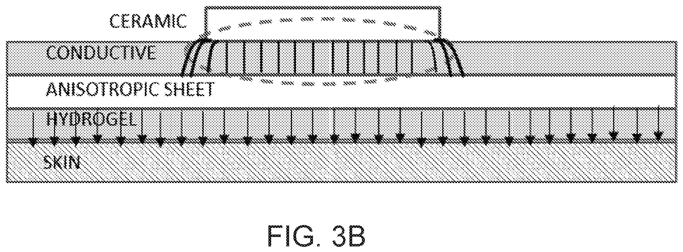

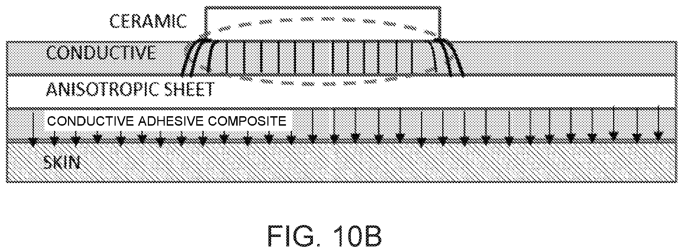

In contrast, FIG. 3B shows the current distribution for the FIG. 3A embodiment. As shown in FIG. 3B, the current is still distributed in the upper hydrogel layer 80 only in the area below the electrode element. However, the sheet of anisotropic material 70 spreads the heat out across its entire area because the thermal conductivity in the horizontal directions (i.e., in directions parallel to the face of the sheet) is much higher than its thermal conductivity in the vertical direction. In addition to spreading out the heat, the low electrical resistance of the sheet 70 in the horizontal direction spreads the current outward throughout the sheet 70, and this spread-out current distribution continues in the front layer of conductive material 60, and thence to the subject's skin. Because the current and heat in this embodiment are both spread out over a larger area of the front layer of conductive material 60, hotspots are eliminated (or at least minimized). This means that for a given applied AC voltage, the hottest point beneath the FIG. 3A/B embodiment will be lower than the hottest point beneath the FIG. 1 prior art example. Accordingly, the current can be increased (with respect to the prior art current) without exceeding the safety temperature threshold at any point beneath the electrode assembly in the FIG. 3A embodiment. And this increase in current will advantageously increase the efficacy of the TTFields treatment. Similar results can be achieved when the hydrogel is replaced with a conductive adhesive composite as disclosed herein (See FIG. 10B).

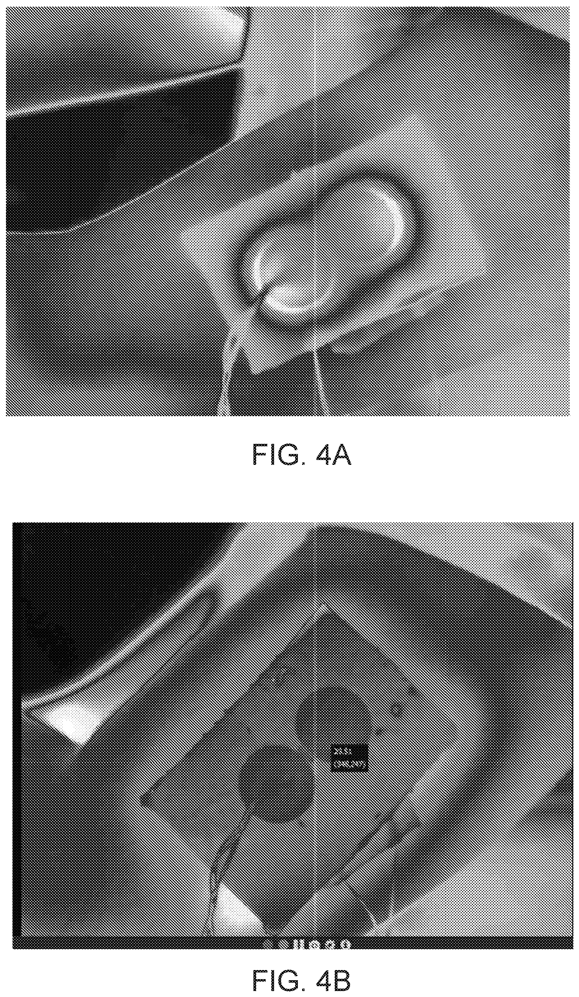

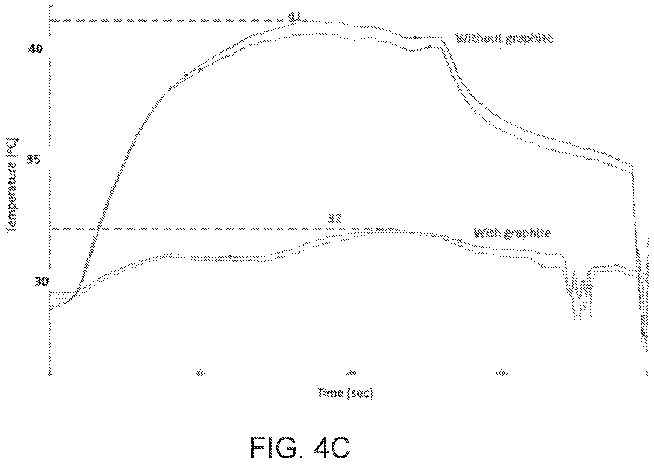

The superior performance of the FIG. 3A embodiment is demonstrated in FIGS. 4A, 4B, and 4C. FIG. 4A is a thermal image of a prior art electrode assembly that includes two electrode elements and a layer of hydrogel disposed on the front faces of the electrode elements (see, e.g., FIG. 1B). There is no sheet of anisotropic material between the front faces of the electrode elements and the rear face of the layer of hydrogel. In use, the front face of the layer of hydrogel is positioned on the subject's skin. FIG. 4A shows hot spots generated in the areas that correspond to the electrode elements.

FIG. 4B is a thermal image of an electrode assembly corresponding to the FIG. 3A embodiment, in which pyrolytic graphite was used as the anisotropic material. FIG. 4B shows that hot spots such as those generated in the prior art electrode assembly have been minimized, and also that the maximum temperature has been reduced. FIG. 4C is a graph comparing the thermal performance of the FIG. 3A embodiment (utilizing pyrolytic graphite as the anisotropic material) with the prior art (no anisotropic material) for the same applied current (500 mA). Notably, the hottest portion of the prior art electrode assembly was 41° C. But when the same 500 mA current was applied to the FIG. 3A embodiment, the hottest portion of the electrode assembly was only 32° C. Similar experiments were performed utilizing graphite foil made from compressed high purity exfoliated mineral graphite as the anisotropic material, with similar results.

In a related experiment, optimized conventional arrays (no anisotropic material), running with 2 A applied current reached the maximum 40 °C average temperature, and were thereby limited. The same type of array with an added pyrolytic graphite sheet (in the manner of the FIG. 3A embodiment) was able to operate at an increased power level (with 3 A applied current), and ran at 38 °C average temperature, 2-3 °C below the temperature threshold limit. This result suggests that the apparatus described herein should be able to achieve more beneficial treatment results by operating at higher applied currents.

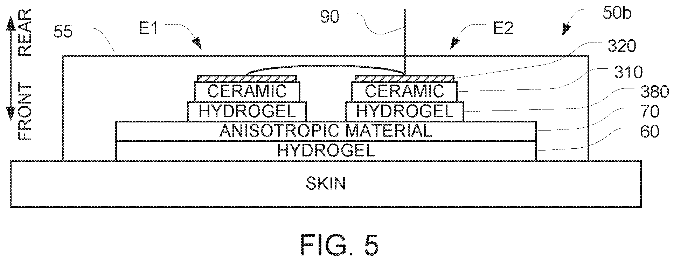

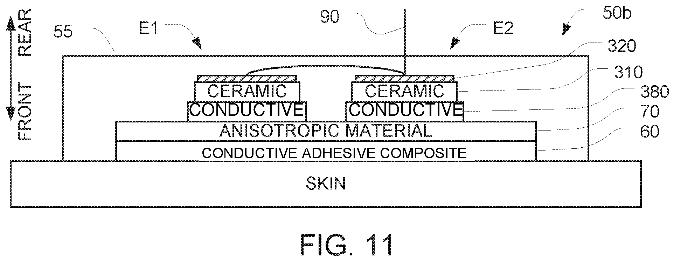

FIG. 5 is a cross sectional representation of a second embodiment of an electrode assembly 50b including electrode elements E1, E2, taken along the dashed line in FIG. 2. The FIG. 5 embodiment is similar to the FIG. 3A embodiment in all respects (including the figure labeling) except as follows. The FIG. 3A and FIG. 10A embodiments include a large continuous layer of conductive material 80 (e.g., hydrogel or conductive adhesive composite) spanning across both electrode elements E1, E2, and positioned between the sheet 70 and the front faces of both the first and second electrode elements E1 and E2. In contrast, the FIG. 5 embodiment includes a separate region of conductive material 380 for each individual electrode element. Thus, the FIG. 5 embodiment includes a first layer of conductive material 380 positioned between the first front face of the first electrode element E1 and the rear face of the sheet 70, and also includes a second layer of conductive material 380 positioned between the second front face of the second electrode element E2 and the rear face of the sheet 70. The first and second layers of conductive material 380 facilitate the electrical contact between the respective electrode front faces and the rear face of the sheet 70. In the embodiment illustrated in FIG. 5, the layers of conductive material 380 are layers of hydrogel. But in alternative embodiments, different conductive materials (e.g., conductive grease, conductive adhesives, conductive tape, etc.) could be used. For example, in the embodiment illustrated in FIG. 11, the layers of conductive material 380 can be layers of conductive adhesive composite as disclosed herein. In some embodiments, the collective area of all the electrode elements is less than the area of the sheet 70, less than half the area of the sheet 70, less than one quarter the area of the sheet 70, or less than one tenth the area of the sheet 70.

As in the FIG. 3A embodiment, the current in the FIG. 5 embodiment is still concentrated in the upper layers of conductive material 380 only in the areas below the electrode elements. The sheet of anisotropic material 70 spreads out the heat and the current as described above in connection with the FIG. 3A embodiment, which eliminates or at least minimizes hot spots. This means that for a given applied AC voltage, the hottest point beneath the electrode assembly in the FIG. 5 embodiment will be at a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 5 embodiment. And this increase in current will advantageously increase the efficacy of the TTFields treatment. Other components of the FIG. 5 construct (e.g., metal layers 320, dielectric material 310, skin contact layer 60, the lead 90 supplying the AC source, and the self-adhesive backing 55) are analogous to those described above for the FIG 3A embodiment.

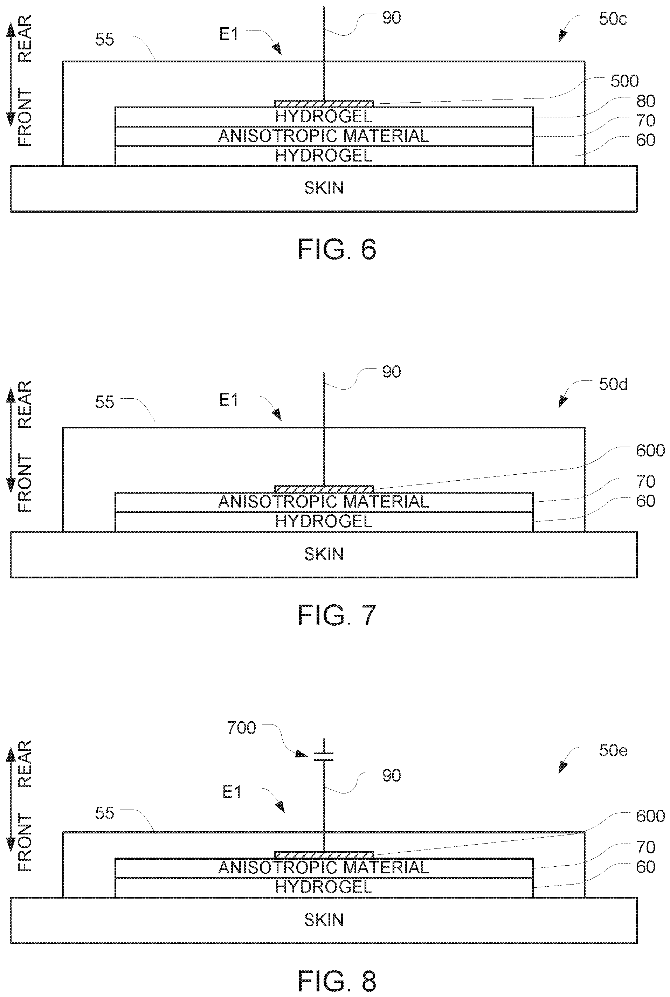

FIG. 6 is a cross sectional representation of a third embodiment of an electrode assembly 50c that includes a single electrode element E1. The embodiment of FIG. 6 is similar to the embodiment of FIG. 3A, except the FIG. 6 embodiment does not include the layer of dielectric material. In the FIG. 6 embodiment, the electrode assembly 50c includes a sheet of anisotropic material 70 having a front face (facing towards the subject's skin in FIG. 6) and a rear face. This sheet 70 is similar to the sheet 70 described above in connection with FIG. 3A. In some embodiments, the sheet of anisotropic material 70 is a sheet of synthetic graphite. In some embodiments, the sheet of anisotropic material 70 is a sheet of pyrolytic graphite. In other embodiments, the sheet of anisotropic material 70 is graphite foil made from compressed high purity exfoliated mineral graphite (e.g., MinGraph^{®} 2010A Flexible Graphite). In other embodiments, the sheet of anisotropic material 70 is a sheet of graphitized polymer film. In other embodiments, the sheet of anisotropic material 70 is a sheet of pyrolytic carbon. In other embodiments, the anisotropic material may be boron nitride. In other embodiments, the sheet of anisotropic material 70 is a sheet of another conductive anisotropic material. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

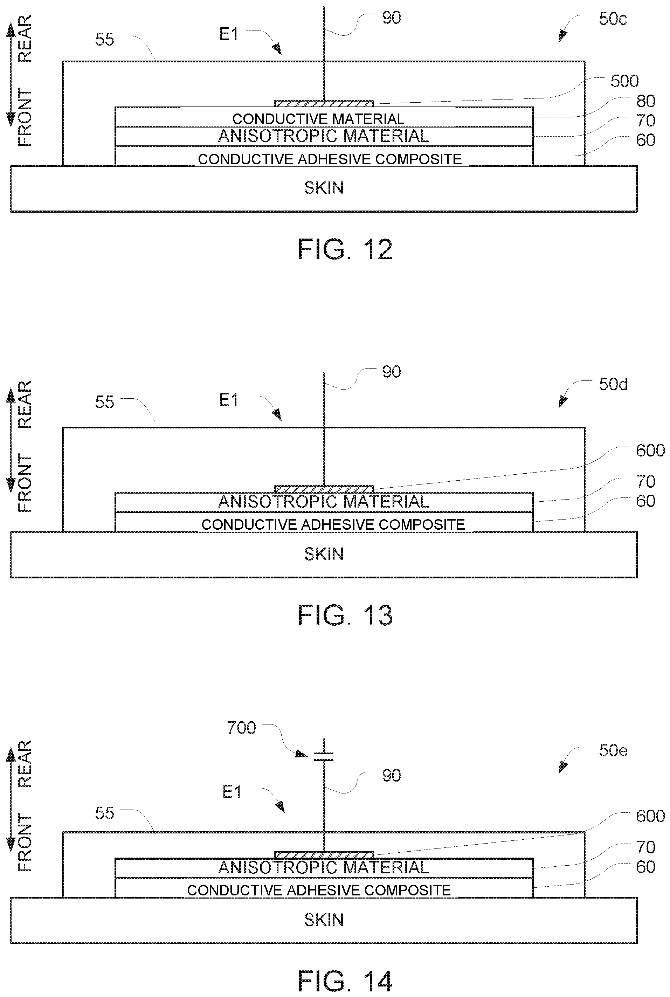

The electrode assembly 50c further includes a front layer of biocompatible conductive material 60 (or skin contact structure 61 (FIGS. 17-21)) disposed on the front face of the sheet 70. Note that in the embodiment illustrated in FIG. 6, there is only a single layer of conductive material 60, and that single layer is biocompatible. But in alternative embodiments described herein there can be more than one layer. Optionally, in such embodiments, only the front layer is biocompatible. The front layer of conductive material 60 (or skin contact structure 61 (FIGS. 17-21)) is configured to ensure good electrical contact between the device and the body. In a preferred embodiment, the front layer of conductive material 60 should cover the entire front face of the sheet of anisotropic material 70. The front layer of conductive material 60 may be the same size or larger (i.e., cover the same area or larger) than the sheet of anisotropic material 70. In some embodiments, the front layer of conductive material 60 (or skin contact structure 61 (FIGS. 17-21)) comprises hydrogel. In these embodiments, the hydrogel may have a thickness between 50 and 2000 µm. In other embodiments, and as shown in FIG. 12, the front layer of conductive material 60 (or skin contact structure 61 (FIGS. 17-21)) comprises a conductive adhesive composite as further disclosed herein. In these embodiments, the biocompatible conductive adhesive may have a thickness between 10 and 2,000 µm, such as, from 20 to 1000 µm, or even 30 to 400 µm.

The electrode assembly 50c further includes a first electrode element E1 positioned behind the sheet 70. The first electrode element E1 includes a piece of metal 500 having a front face disposed in electrical contact with the rear face of the sheet 70. In the FIG. 6 embodiment, the front face of the piece of metal 500 is the first front face of the first electrode element E1. Accordingly, the FIG. 6 embodiment differs from the FIG. 3A or FIG. 5 embodiments by omitting a layer of dielectric material. The positional relationship between the first electrode element E1 and the sheet 70 in this FIG. 6 embodiment may be as described above in connection with FIG. 3A.

The electrode assembly 50c further includes a first layer of conductive material 80 positioned between the first front face of the first electrode element E1 (i.e., the front face of the piece of metal 500) and the rear face of the sheet 70. The first layer of conductive material 80 facilitates the electrical contact between the first front face of the first electrode element E1 and the rear face of the sheet 70. In the illustrated embodiment, the layer of conductive material 80 is a layer of hydrogel. But in alternative embodiments, a different conductive material (e.g., conductive grease, conductive adhesives, conductive tape, etc.) could be used. For example, in FIG. 12, the layer of conductive material 80 can comprise a conductive adhesive composite as further disclosed herein.

The piece of metal 500 of the electrode element E1 is wired (e.g., using wires, traces on a flex circuit, etc.) to a lead 90, which supplies an AC voltage from an AC voltage generator (not shown) to the electrode element to generate the TTFields when the electrode assembly 50c is affixed to the subject's body for treatment.

The electrode assembly 50c may optionally include one or more additional electrode elements (not shown) having a structure identical to electrode element E1 and positioned to have the same functionality. In such case, the pieces of metal 500 of all the electrode elements may be wired together (e.g., using wires, traces on a flex circuit, etc.) to the lead 90.

In some embodiments that include only a single electrode element E1, the area of the sheet 70 is larger (e.g., at least 2 times larger, at least 4 times larger, or at least 10 times larger) than the area of the electrode element E1. In some embodiments that include a plurality of electrode elements (not shown) the area of the sheet 70 is larger (e.g., at least 2, 4, or 10 times larger) than the collective area of all of the electrode elements. When an AC voltage is applied to the electrode elements, the heat spreads out across the entire sheet 70, which minimizes or eliminates hot spots.

Similar to the FIG. 3A embodiment, the sheet of anisotropic material 70 in the FIG. 6 embodiment spreads out the heat and the current as described above in connection with the FIG. 3A embodiment, which eliminates or at least minimizes hot spots. This means that for a given applied AC voltage, the hottest point beneath the electrode assembly in the FIG. 6 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 6 embodiment. And this increase in current will advantageously increase the efficacy of the TTFields treatment.

FIG. 7 is a cross sectional representation of a fourth embodiment of an electrode assembly 50d that includes a single electrode element E1. The FIG. 7 embodiment is similar to the FIG. 6 embodiment except that the first front face of the first electrode element E1 (i.e., the front face of the piece of metal 600) is positioned in direct contact with the rear face of the sheet 70 (instead of being electrically connected via an intervening layer of conductive material).

Similar to the FIG. 6 embodiment, the sheet of anisotropic material 70 in the FIG. 7 embodiment spreads out the heat and the current as described above in connection with the FIG. 3A embodiment, which eliminates or at least minimizes hot spots. This means that for a given applied AC voltage, the hottest point beneath the electrode assembly in the FIG. 7 embodiment will be lower than the hottest point beneath the electrode assembly in the FIG. 1 prior art example. Accordingly, the current can be increased (with respect to the prior art current) without exceeding the safety temperature threshold at any point beneath the electrode assembly in the FIG. 7 embodiment. And this increase in current will advantageously increase the efficacy of the TTFields treatment.

FIG. 8 is a cross sectional representation of a fifth embodiment of an electrode assembly 50e that includes a single electrode element E1. The FIG. 8 embodiment is similar to the FIG. 7, but it adds a capacitor 700 connected in series with and behind the piece of metal 600. A similar addition of a capacitor 700 connected in series with and behind the piece of metal 500 could also be envisioned for the FIG. 6 embodiment.

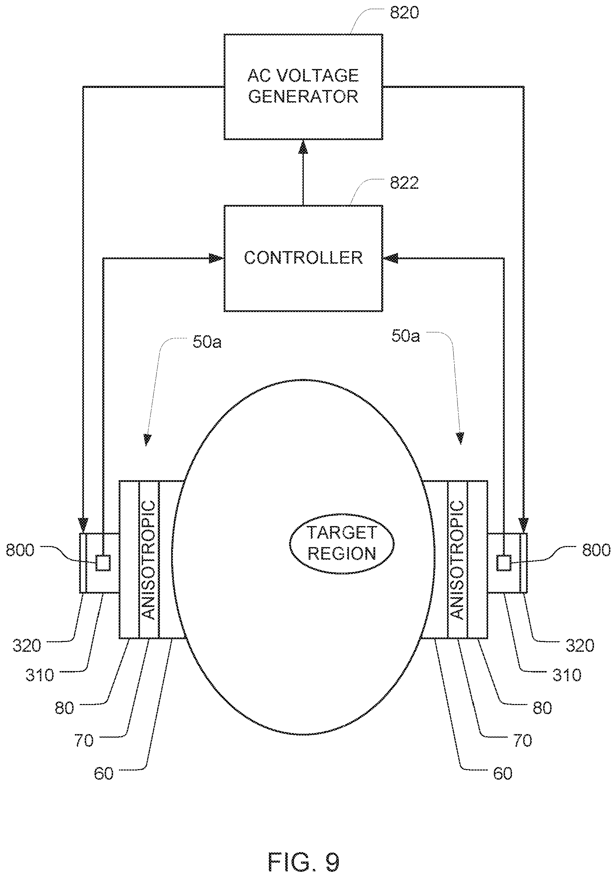

FIG. 9 shows how a pair of the FIG. 3A electrode assemblies 50a may be used to apply an alternating electric field to a target region in the subject's body. The subject can be a human or another mammal, including but not limited to rats and mice. (Note that any of the electrode assemblies described above in connection with FIGS. 5-8 may be used instead of the FIG. 3A electrode assemblies 50a shown here).

The exemplary method includes positioning a first electrode assembly 50a at a first position on or in the subject's body. (In the example depicted in FIG. 9, the first electrode assembly 50a is positioned on the subject's skin at the right of the subject's head facing a target region, e.g., a tumor.) The first electrode assembly 50a may be constructed as described earlier herein. In the FIG. 9 embodiment, the first electrode assembly 50a includes a first sheet 70 of anisotropic material having a first front face and a first rear face. The first sheet 70 has a first thermal conductivity in a direction that is perpendicular to the first front face. Thermal conductivity of the first sheet 70 in directions that are parallel to the first front face of the sheet 70 is more than two times higher than the first thermal conductivity. The first sheet 70 has a first resistance in a direction that is perpendicular to the front face, and the resistance of the first sheet in directions that are parallel to the front face is less than half of the first resistance. During use, the first electrode assembly 50a is positioned so that the first front face of the first sheet 70 faces the target region.

The exemplary method also includes positioning a second electrode assembly 50a at a second position in or on the subject's body. (In the example depicted in FIG. 9, the second electrode assembly 50a is positioned on the subject's skin at the left of the subject's head facing the target region.) The second electrode assembly 50a may be constructed as described earlier herein. In the FIG. 9 embodiment, the second electrode assembly 50a includes a second sheet 70 of anisotropic material 70 having a second front face and a second rear face. The second sheet 70 has a second thermal conductivity in a direction that is perpendicular to the second front face. Thermal conductivity of the second sheet 70 in directions that are parallel to the second front face of the sheet 70 is more than two times higher than the second thermal conductivity. The second sheet 70 has a second resistance in a direction that is perpendicular to the front face, and the resistance of the second sheet in directions that are parallel to the front face is less than half of the second resistance. During use, the second electrode assembly 50a is positioned so that the second front face of the second sheet 70 faces the target region.

The exemplary method further includes applying an alternating voltage between the first electrode assembly 50a and the second electrode assembly 50a. The applying is performed after positioning the first electrode assembly 50a and the second electrode assembly 50a. The applying may be implemented by applying the alternating voltage between (i) a first electrode element disposed in electrical contact with the first rear face of the first sheet 70 and (ii) a second electrode element disposed in electrical contact with the second rear face of the second sheet 70.

In some embodiments, the first electrode assembly 50a further includes a first layer of biocompatible conductive material 60 disposed on the first front face of the first sheet 70. Correspondingly, the second electrode assembly further includes a second layer of biocompatible conductive material 60 disposed on the second front face of the second sheet 70. As described above, the biocompatible conductive material 60 may be hydrogel or may be a conductive grease, conductive adhesive including the non-hydrogel conductive adhesives discussed above, conductive tape, conductive composite, etc.

In some embodiments, the first electrode assembly 50a further includes a first rear layer of conductive material 80 (as described above) positioned between the first front face (skin-facing surface) of the first electrode element of the first electrode assembly 50a and the first rear face (outwardly facing surface) of the first sheet 70. Correspondingly, the second electrode assembly further includes a second rear layer of conductive material 80 (as described above) positioned between the second front face of the second electrode element of the second electrode assembly and the second rear face of the second sheet 70.

In some embodiments, each of the first and second sheets of anisotropic material 70 is a sheet of synthetic graphite. In some embodiments, the sheet of anisotropic material 70 is a sheet of pyrolytic graphite. In other embodiments, each of the first and second sheets of anisotropic material 70 is graphite foil made from compressed high purity exfoliated mineral graphite or a sheet of graphitized polymer film. In other embodiments, the anisotropic material may be pyrolytic carbon. In other embodiments, the anisotropic material may be boron nitride. Other embodiments may utilize sheets of other conducting materials with anisotropic properties. In some embodiments (e.g., when the sheets of anisotropic material are sheets of synthetic graphite, such as pyrolytic graphite or compressed high purity exfoliated mineral graphite or graphitized polymer film), the sheets of anisotropic material 70 are nonmetallic.

The alternating voltage between the first electrode assembly and the second electrode assembly may be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 50a in order to maintain temperatures below a safety threshold (e.g., 41° C). This may be accomplished, for example, by measuring a first temperature of the first electrode element, measuring a second temperature of the second electrode element, and controlling the applying of the alternating voltage based on the first temperature and the second temperature, as described below.

FIG. 9 depicts one example of hardware that is suitable for this purpose. More specifically, temperature sensors 800 (e.g., thermistors) are positioned in thermal contact with respective electrode elements (for example, dielectric material 310 / layer of metal 320) within each of the electrode assemblies 50a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Similar embodiments and methods are envisaged utilizing any of the electrode assemblies 50a-e, or combinations thereof, in place of either or both of the first electrode assembly 50a and the second electrode assembly 50a.

### Electrode Assemblies Having Skin Contact Layers that Comprise a Conductive

### Adhesive Composite

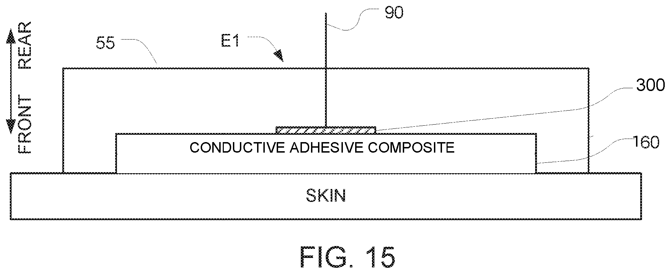

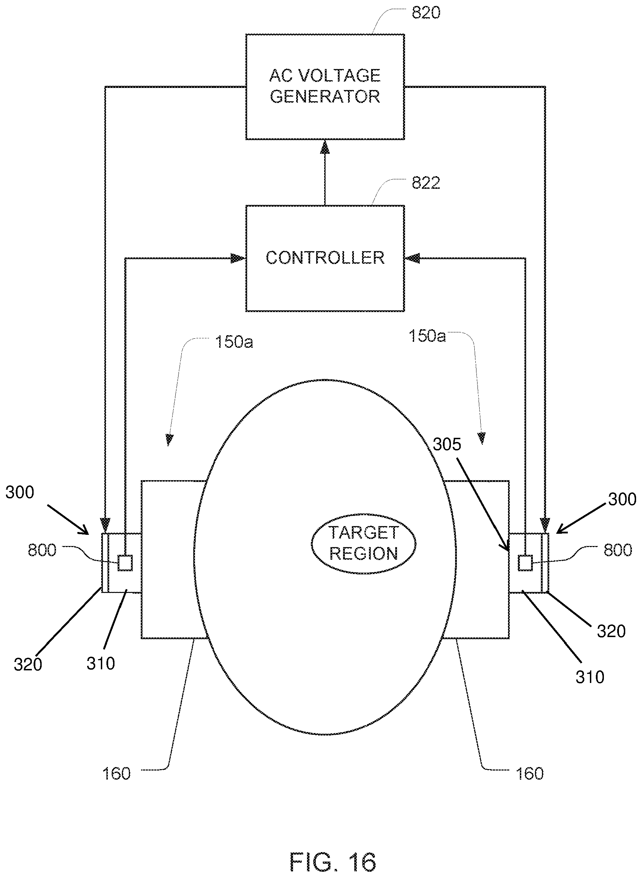

As discussed above, it is contemplated that one or more of the layers of conductive materials 60, 80, 380 (or the skin contact structure 61 disclosed herein with reference to FIGS. 17-21) can comprise conductive adhesive composites (described further below) rather than hydrogel. Exemplary electrode assemblies including such conductive adhesive composites are depicted in FIGS. 10A-14, which depict the embodiments of FIGS. 3A-3B and 5-8 with conductive adhesive composite used in place of hydrogel as a skin contact layer, and in FIG. 15, which includes a skin contact layer comprising a conductive adhesive composite but does not include a layer of anisotropic material. In other aspects, and as depicted in FIG. 16, it is contemplated that electrode assemblies comprising conductive adhesive composites can be used to apply TTFields. In other respects, the FIG. 16 embodiments may include similar electrode assembly features and components as described above for the FIG. 9 embodiments.

In exemplary aspects, and with reference to FIG. 16, a exemplary method can comprise positioning at least first and second electrode assemblies 150a on a body of a subject. In these aspects, each of the first and second electrode assemblies can comprise at least one electrode element 300 having a skin-facing surface 305. As used herein, the term "skin-facing" refers to a direction or orientation that would be toward the skin of a patient when used as disclosed herein (for example, to apply TTFields with the electrode assembly adhered to the skin of a subject). Optionally, each electrode element 300 may comprise a dielectric component (e.g., a ceramic), not shown in FIG. 16. However, in some aspects, such dielectric components (e.g., ceramics) can be omitted. Generally, it should be understood that the first and second electrode assemblies 150a can be embodied by any of the electrode assemblies described herein, including the embodiments with reference to FIGS. 3A, 3B, 5-8, 10A-15, and 17-21.

Each of the first and second electrode assemblies can further comprise a skin contact layer 160 comprising a conductive adhesive composite. As used herein, the term "skin contact layer" refers to a layer that is configured to contact the skin of a subject when used as disclosed herein (for example, to apply TTFields with the electrode assembly adhered to the skin of the subject). In exemplary aspects, the conductive adhesive composite can comprise a dielectric material and conductive particles dispersed within the dielectric material. In some embodiments, at least a portion of the conductive particles define a conductive pathway through a thickness of the conductive adhesive composite. It is contemplated that the conductive particles can be aligned in response to application of an electric field such that the conductive particles undergo electrophoresis. In some aspects, the dielectric material of the conductive adhesive composite of each of the first and second electrode assemblies is a polymeric adhesive. Optionally, in these aspects, the polymeric adhesive can be an acrylic adhesive. In some aspects, the conductive particles can comprise carbon. Optionally, in these aspects, the conductive particles can comprise graphite powder. Additionally, or alternatively, the conductive particles can comprise carbon flakes. Additionally, or alternatively, the conductive particles can comprise carbon granules. Additionally, or alternatively, the conductive particles can comprise carbon fibers. Additionally, or alternatively, the conductive particles can comprise carbon nanotubes or carbon nanowires. Additionally, or alternatively, the conductive particles can comprise carbon black powder. In further aspects, the conductive adhesive composite further comprises a polar material (e.g., a polar salt). The polar salt may be a quaternary ammonium salt, such as a tetra alkyl ammonium salt. Exemplary conductive adhesive composites, as well as methods for making such conductive adhesive composites, are disclosed in U.S. Patent No. 8,673,184 and U.S. Patent No. 9,947,432. In exemplary aspects, the conductive adhesive composite can be a dry carbon/salt adhesive, such as the OMNI-WAVE adhesive compositions, described above, manufactured and sold by FLEXCON (Spencer, MA, USA).

In further aspects, the at least one electrode element 300 can be electrically coupled to (optionally, in electrical contact with) the skin contact layer 160, and the method can further comprise applying an alternating voltage between the first electrode assembly and the second electrode assembly, thereby generating an electric field (e.g., TTFields).

As discussed above with respect to FIG. 9, FIG. 16 depicts temperature sensors 800 (e.g., thermistors) positioned in thermal contact with respective electrode elements (for example, dielectric material 310 / layer of metal 320) within each of the electrode assemblies 150a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Optionally, as shown in FIG. 16, the skin-facing surface 305 of the at least one electrode element 300 of the first electrode assembly can be in electrical contact with the skin contact layer 160 (or skin contact structure 61 (FIGS. 17-21)) of the first electrode assembly. Similarly, it is contemplated that the skin-facing surface of the at least one electrode element of the second electrode assembly can be in electrical contact with the skin contact layer of the second electrode assembly.

Optionally, as shown in FIGS. 9 and 10A-14, it is contemplated that the first electrode assembly of the FIG. 16 embodiment can further comprise a layer of anisotropic material 70 having a skin-facing surface and an opposing outwardly facing surface (not shown). In these aspects, the at least one electrode element 300 of the first electrode assembly can be electrically coupled to (optionally, in electrical contact with) the outwardly facing surface of the layer of anisotropic material 70, and the skin contact layer 160 (or skin contact structure 61 (FIGS. 17-21)) of the first electrode assembly can be disposed on the skin-facing side of the layer of anisotropic material, optionally, on the skin-facing surface of the layer of anisotropic material. Similarly, it is contemplated that the second electrode assembly can likewise comprise a layer of anisotropic material as disclosed with respect to the first electrode assembly. As shown in FIGS. 9 and 10A-12, in some aspects, it is contemplated that each electrode assembly can optionally comprise a conductive layer 80 positioned between the electrode elements and the anisotropic material 70. In these aspects, it is contemplated that the material of the conductive layer 80 can optionally be a conductive adhesive composite as disclosed herein. Alternatively, it is contemplated that the conductive layer 80 can be another conductive material as further disclosed herein.

In exemplary aspects, the skin contact layer 160 (or skin contact structure 61 (FIGS. 17-21)) of each of the first and second electrode assemblies does not comprise hydrogel.

In further exemplary aspects, the skin contact layer 160 (or skin contact structure 61 (FIGS. 17-21)) of each of the first and second electrode assemblies does not comprise a latex rubber polymer.

In further exemplary aspects, the skin contact layer of each of the first and second electrode assemblies does not comprise silver of silver chloride.

In still further aspects, the conductive adhesive composite of each of the first and second electrode assemblies has a thickness ranging from about 30 µm to about 2000 µm, such as from 30 µm to about 70 µm. Optionally, the conductive adhesive composite can have a thickness ranging from about 45 µm to about 55 µm

In still further aspects, the conductive adhesive composite of each of the first and second electrode assemblies does not comprise water.

In exemplary aspects, the conductive particles of the conductive adhesive composite comprise a plurality of groups of conductive particles. In some embodiments, the conductive particles of the collective plurality of groups of conductive particles are aligned to define a conductive pathway through the thickness of the conductive adhesive composite of each of the first and second electrode assemblies.

In further exemplary aspects, and as disclosed above, an apparatus can comprise: at least one electrode element having a skin-facing surface; a layer of anisotropic material having a skin-facing surface and an opposing outwardly facing surface; and a skin contact layer comprising a conductive adhesive composite. In these aspects, the at least one electrode element can be electrically coupled to (optionally, in electrical contact with) the outwardly facing surface of the layer of anisotropic material, and the skin contact layer can be disposed on the skin-facing side of the layer of anisotropic material, optionally, on the skin-facing surface of the layer of anisotropic material.

Optionally, in exemplary aspects, the skin contact layer can be releasably connected to the layer of anisotropic material. In these aspects, it is contemplated that the skin contact layer can be selectively detached from the anisotropic material and replaced with a new skin contact layer (for example, when a maximum/threshold duration of use is approached or met).

In exemplary aspects, the sheet of anisotropic material is a synthetic graphite.

In exemplary aspects, the sheet of anisotropic material is a sheet of pyrolytic graphite or graphite foil made from compressed high purity exfoliated mineral graphite or graphitized polymer film.

In exemplary aspects, the sheet of anisotropic material is nonmetallic.

In exemplary aspects, the sheet of anisotropic material has a first thermal conductivity in a direction that is perpendicular to the plane of the sheet, and thermal conductivity of the sheet in directions that are parallel to the plane of the sheet is more than two times higher, or, even, 10 times higher than the first thermal conductivity.

In exemplary aspects, the sheet of anisotropic material has a first resistance in a direction that is perpendicular to the plane of the sheet, and wherein resistance of the sheet in directions that are parallel to the plane of the sheet is less than half, or, even, less than 10% of the first resistance.

Optionally, in exemplary aspects, the apparatus can further comprise a release liner that covers the skin contact layer. In these aspects, it is contemplated that, prior to use, the apparatus can be provided with the release liner to ensure that the skin contact layer does not adhere to undesirable surfaces or locations. Immediately prior to use, the release liner can be removed, and the skin contact layer can be positioned in contact with the skin of the patient.

In exemplary aspects, by using a conductive adhesive composite as a skin contact layer as disclosed herein, it is contemplated that additional backing and/or cover layers (such as, for example self-adhesive backing 55) can, optionally, be omitted. In these aspects, it is contemplated that the conductive adhesive composite can provide sufficient adhesion to the skin such that it is unnecessary to provide additional layers to maintain a desired position of the electrode assembly on the body of the subject, thereby improving ease of use and decreasing the overall cost of manufacture and use.

In further aspects, by avoiding the use of hydrogel within an electrode assembly, it is contemplated that electrode assemblies comprising conductive adhesive composites as disclosed herein do not require moisture barrier packaging, thereby making the cost of packaging far more affordable. Additionally, it is contemplated that the conductive adhesive composites of the disclosed electrode assemblies can avoid the signal variation issues of hydrogels, thereby providing consistent material properties (e.g., tackiness) and reliable performance during delivery of TTFields. Further, it is contemplated that the disclosed conductive adhesive composites can have a far greater shelf life than hydrogels, thereby decreasing the frequency at which electrode assemblies (or the skin contact layers of electrode assemblies) must be replaced.

It is further contemplated that embodiments that include the sheet of anisotropic material may additionally aid in avoiding or reducing overheating of the electrodes and associated discomfort on the skin by dissipating both electrical current and heat in a lateral (in-plane) direction rather than passing directly through the layer (in a direction perpendicular to the plane of the skin contact layer) in a concentrated manner.

### Electrode Assemblies Having Skin Contact Structures that Comprise Multiple Adhesive Layers

Referring to FIGS. 17-21, in some aspects, the front (skin-facing) layer of conductive material 60 (of FIG. 3A) can be embodied as a skin contact structure 61. Referring to FIG. 17, the electrode assembly 50a includes a layer of anisotropic material 70 (referred to herein also as a sheet of anisotropic material) having a (front) skin-facing surface 72 (facing towards the subject's skin in FIG. 17) and an opposing outwardly facing (rear) surface 74. This sheet 70 has a first thermal conductivity in a direction that is perpendicular to the front face. Thermal conductivity of the sheet 70 in directions that are parallel to the front face is more than two times higher than the first thermal conductivity. In some preferred embodiments, the thermal conductivity of the sheet 70 in directions that are parallel to the front face is more than ten times higher than the first thermal conductivity. The sheet 70 in the FIG. 17 embodiment is also anisotropic in another respect. More specifically, the sheet 70 has a first resistance in a direction that is perpendicular to the front face, and the resistance of the sheet in directions that are parallel to the front face is less than half of the first resistance. In some embodiments, the resistance of the sheet in directions that are parallel to the front face is less than 10% of the first resistance. Each electrode element (e.g., electrode elements E1, E2) can be in electrical contact with the outwardly facing surface 74 of the layer of anisotropic material 70.

The electrode assembly 50a further includes a skin contact structure 61 disposed on the front face of the sheet 70. The skin contact structure 61 is configured to ensure good electrical contact between the device and the body. In some embodiments, the skin contact structure 61 can cover the entire front face of the sheet of anisotropic material 70. The skin contact structure 61 may have an areal footprint the same size or larger than the sheet of anisotropic material 70. In some embodiments, the skin contact structure 61 comprises hydrogel. In these embodiments, the hydrogel may have a thickness between 50 and 2000 µm. In other embodiments, the skin contact structure 61 comprises a conductive adhesive composite as further disclosed herein.

The electrode assembly 50a further includes a first electrode element E1 positioned behind the sheet 70. The first electrode element E1 has a first front face disposed in electrical contact with the rear face of the sheet 70. In the FIG. 17 embodiment, the first electrode element E1 includes a first layer of dielectric (e.g., ceramic) material 310 having a front face and a rear face, and a first layer of metal 320 disposed on the rear face of the first layer of dielectric material 310. The front face of the first layer of dielectric material 310 is the first front face of the first electrode element E1. The first layer of dielectric material 310 can be disposed on an outwardly facing side 78 of the layer of anisotropic material 70. Optionally, the first layer of dielectric material 310 can have a dielectric constant of at least 10.

The electrode assembly 50a may optionally include one or more additional electrode elements. In the illustrated embodiment, the electrode assembly 50a includes a second electrode element E2 positioned behind the sheet 70. The second electrode element E2 has a second front face disposed in electrical contact with the rear face of the sheet 70. The two electrode elements E1, E2 in FIG. 17 have identical structures. Thus, the second electrode element E2 includes a second layer of dielectric (e.g., ceramic) material 310 having a front face and a rear face, and a second layer of metal 320 disposed on the rear face of the second layer of dielectric material 310. The front face of the second layer of dielectric material 310 is the second front face of the second electrode element E2. Each electrode element can have a skin-facing surface 52.

The electrode assembly 50a further includes a first layer of conductive material 80 positioned between the first front face of the first electrode element E1 (i.e., the front face (skin-facing surface) of the first layer of dielectric material 310) and the rear face of the sheet 70. The first layer of conductive material 80 facilitates the electrical contact between the first front face of the first electrode element E1 and the rear face of the sheet 70. In the illustrated embodiment, the layer of conductive material 80 can be a layer of hydrogel. In alternative embodiments, a different conductive material (e.g., conductive grease, conductive adhesives, conductive tape, etc.) can be used. For example, in FIG. 17, the layer of conductive material 80 can comprise a conductive adhesive composite as further disclosed herein.

The first layer of conductive material 80 (e.g., an upper adhesive layer) is also positioned between the second front face of the second electrode element E2 (i.e., the front face of the second layer of dielectric material 310) and the rear face of the sheet 70. The first layer of conductive material 80 facilitates the electrical contact between the second front face of the second electrode element E2 and the rear face of the sheet 70. As described for E1, and as shown in FIG. 3A, the conductive material 80 may be a layer of hydrogel, but in alternative embodiments, a different conductive material may be used (e.g., conductive grease, conductive adhesives, conductive tape, etc.). For example, in FIG. 17, the layer of conductive material 80 can comprise a conductive adhesive composite as further disclosed herein.

As illustrated in FIG. 17, the skin contact structure 61 can have an outer adhesive layer 62, an inner adhesive layer 64, and a substrate 66 positioned between the outer and inner adhesive layers 62, 64. Although shown in FIGS. 18-21 as a single component, it is contemplated that the skin contact structure 61 depicted in these figures can include the inner and outer adhesive layers and substrate as shown in FIG. 17. The outer adhesive layer 62 can be disposed on a skin-facing side 76 of the layer of anisotropic material 70). The inner adhesive layer 64 can be configured to contact skin of a subject. Optionally, the outer adhesive layer 62 of the skin contact structure 61 can be disposed on the skin-facing surface 72 of the layer of anisotropic material.

Referring to FIG. 17, in some aspects, the outer and inner adhesive layers 62, 64 of the skin contact structure 61 do not comprise hydrogel. For example, the outer and inner adhesive layers 62, 64 of the skin contact structure 61 can comprise conductive adhesive composite, as further disclosed herein. Alternatively, it is contemplated that the outer and/or inner adhesive layers 62, 64 can comprise hydrogel.

In some optional aspects, the outer adhesive layer 62 and/or the inner adhesive layer 64 can comprise carbon black. For example, in exemplary aspects, the outer adhesive layer 62 and/or the inner adhesive layer 64 can comprise adhesive provided by ADHESIVE RESEARCH, such as ARcare^{®} 8006 electrically conductive adhesive composition manufactured and sold by Adhesives Research, Inc. (Glen Rock, PA, USA). In other optional aspects, the outer adhesive layer 62 and/or the inner adhesive layer 64 can comprise carbon fibers or nanowires. For example, in exemplary aspects, the outer adhesive layer 62 and/or the inner adhesive layer 64 can comprise a dry carbon/salt adhesive, such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon.

In some optional aspects, the outer adhesive layer 62 can have a thickness of at least 40 µm (optionally, at least 45 µm or at least 50 µm). For example, the thickness of the outer adhesive layer 62 can range from about 40 µm to about 75 µm (e.g., optionally, from about 40 µm to about 70 µm, or from about 40 µm to about 65 µm, or from about 45 µm to about 75 µm, or from about 45 µm to about 70 µm, or from about 45 µm to about 65 µm or from about 50 µm to about 75 µm or from about 50 µm to about 70 µm, or from about 50 µm to about 65 µm). In further optional aspects, the inner adhesive layer 64 can have a thickness of at least 40 µm (optionally, at least 45 µm or at least 50 µm). For example, the thickness of the inner adhesive layer 64 can range from about 40 µm to about 75 µm (e.g., optionally, from about 40 µm to about 70 µm, or from about 40 µm to about 65 µm, or from about 45 µm to about 75 µm, or from about 45 µm to about 70 µm, or from about 45 µm to about 65 µm or from about 50 µm to about 75 µm or from about 50 µm to about 70 µm, or from about 50 µm to about 65 µm). It was discovered that an excessive thickness (e.g., greater than 75 µm or, even, greater than 65 µm in certain embodiments) can leave a residue following removal from a subject or patient. It was further discovered that an insufficient thickness (e.g., less than 25 µm in certain embodiments) can result in the layers breaking too easily.

In some embodiments, the substrate 66 can be electrically conductive. In various optional aspects, the substrate 66 of the skin contact structure 61 can have a continuous, uninterrupted structure. In these aspects, it is contemplated that the substrate can be electrically conductive to conduct electricity between the outer and inner adhesive layers 62, 64, in like manner to scanning electron microscopy (SEM) tape.

In alternative aspects, the substrate 66 of the skin contact structure 61 can have an at least partially open structure that is configured to permit flow of adhesive between or among the inner and outer adhesive layers 64, 62 of the skin contact structure. In this way, the adhesive can conduct electricity through the substrate 66. For example, in some aspects, the substrate 66 can comprise a mesh. Optionally, the mesh can have a density from about 6 grams per square meter to about 8 grams per square meter. In other aspects, the substrate 66 can comprise a scrim.

In various optional aspects, the substrate 66 of the skin contact structure 61 can comprise paper or any suitable polymer (e.g., polyester, polyolefin, etc.).

Optionally, the skin contact structure 61 can be reusable.

In embodiments in which the inner and/or outer adhesive layers of the skin contact structure comprise a conductive adhesive composite, the conductive adhesive composite of the inner and outer adhesive layers of the skin contact structure can comprise a dielectric material; and conductive particles dispersed within the dielectric material. Optionally, the conductive particles comprise a plurality of groups of conductive particles. In some optional aspects, the dielectric material of the conductive adhesive composite can be a polymeric adhesive, such as an acrylic polymer. Conductive particles may include, for example, carbon particles, such as graphite powder, carbon flakes, carbon granules, carbon fibers, carbon nanotubes, carbon nanowires, or carbon black powder.

As skin contact layers, both hydrogel and, to a lesser degree, conductive adhesive composites are subject to transfer of adhesive to the subject's skin when the array is removed or repositioned. Importantly, the use of a central substrate in a 3-layer skin contact structure described herein, and particularly the use of a scrim or mesh layer as the central substrate, has minimal or no adhesive transfer to the subject's skin upon removal or repositioning.

In some aspects, the layer of anisotropic material 70 has a first thermal conductivity in a direction that is perpendicular to a plane of the layer. Thermal conductivity of the layer of anisotropic material 70 in directions that are parallel to the plane of the layer can be more than two times higher, or, even, 10 times higher than the first thermal conductivity.

In some aspects, the layer of anisotropic material 70 can have a first resistance in a direction that is perpendicular to a plane of the layer. Resistance of the layer in directions that are parallel to the plane of the layer can be less than half the first resistance, or, even, less than 10% of the first resistance.

The layer of anisotropic material 70 can be or can comprise a layer of synthetic graphite. In other embodiments, the layer of anisotropic material 70 can be or can comprise a layer of pyrolytic graphite, graphitized polymer film, or graphite foil made from compressed high purity exfoliated mineral graphite.

Referring to FIGS. 9, 16, and 17, an exemplary method can comprise positioning at least first and second electrode assemblies on a body of a subject. As described herein with reference to FIGS. 17-21, each of the first and second electrode assemblies can comprise at least one electrode element (e.g., E1) having a skin-facing surface 52, a layer of anisotropic material 70 having a skin-facing surface 72 and an opposing outwardly facing surface 74, and a skin contact structure 61. The skin contact structure 61 can comprise an outer adhesive layer 62 (optionally, comprising conductive adhesive composite); an inner adhesive layer 64 (optionally, comprising conductive adhesive composite); and a substrate 66 positioned between the inner and outer adhesive layers (which, optionally, may be a mesh or scrim). The at least one electrode element can be in electrical contact with the outwardly facing surface of the layer of anisotropic material. The outer adhesive layer 62 of the skin contact structure 61 can be disposed on the skin-facing side 76 of the layer of anisotropic material, and, optionally, may be disposed on the skin-facing surface 72 of the layer of anisotropic material. The inner adhesive layer 64 of the skin contact structure 61 can contact skin of the subject. An alternating voltage can be applied between the first electrode assembly and the second electrode assembly, thereby generating an electric field.

Referring also to FIG. 18, contrasting to FIG. 17, in some optional aspects, the conductive material 80 can include a separate region of conductive material 380 for each individual electrode element. Thus, the FIG. 18 embodiment includes a first layer of conductive material 380 positioned between the first front face of the first electrode element E1 and the rear face of the sheet 70, and also includes a second layer of conductive material 380 positioned between the second front face of the second electrode element E2 and the rear face of the sheet 70.

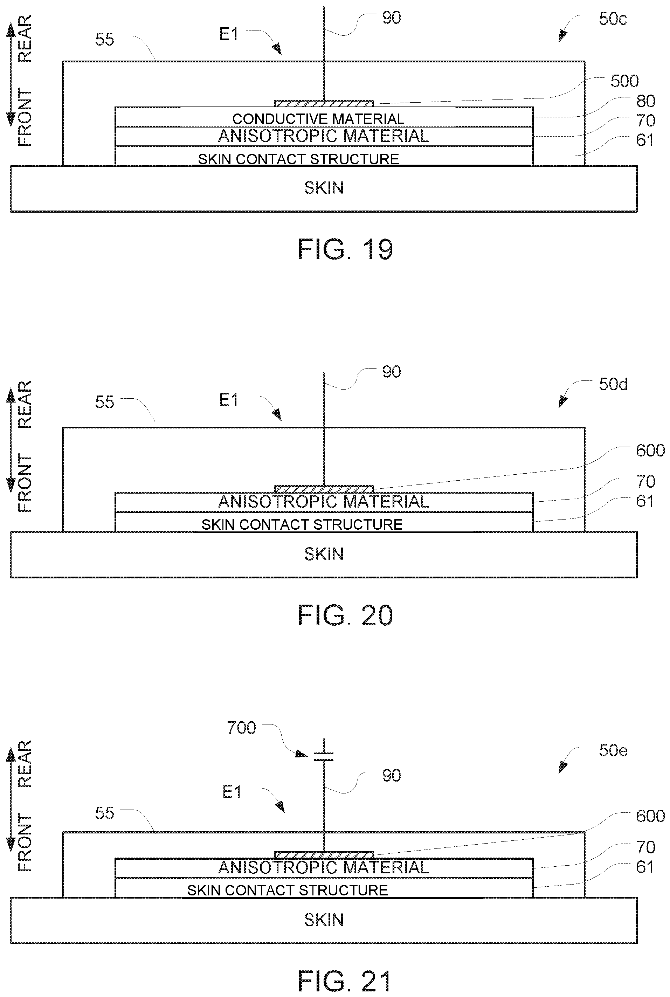

Referring to FIG. 19, optionally, the dielectric material (e.g., ceramic or high dielectric constant polymer) can be omitted. Referring to FIG. 20, optionally, the first layer of conductive material 80 between the piece of metal 600 and the anisotropic material 70 can further be omitted.

Referring to FIG. 21, optionally, the capacitor 700 can be connected in series with and behind the piece of metal 600. For example, the FIG. 21 embodiment is similar to that of FIG. 20, but it includes capacitor 700 connected in series with and behind the piece of metal 600. A similar addition of a capacitor 700 connected in series with and behind the piece of metal 500 could also be envisioned for the FIG. 19 embodiment.

Generally, it is contemplated that aspects disclosed herein with reference to FIGS. 1-16 are also applicable to the embodiments described with reference to FIGS. 17-21. For example, the anisotropic material 70 and conductive materials (e.g., conductive adhesive composites) shown and described with reference to FIGS. 1-16 can be embodied as disclosed herein with reference to FIGS. 17-21. Likewise, aspects disclosed herein with reference to FIGS. 17-21 can be applicable to aspects disclosed with reference to FIGS. 1-16. For example, the front layer of conductive material 60 of any one of FIGS. 1-16 can be embodied as a skin contact structure 61 having inner and outer adhesive layers and a substrate, as shown and described with reference to FIGS. 17-21.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. An apparatus, comprising:
at least one electrode element (E1, E2) having a skin-facing surface;
a layer of anisotropic material (70) having a skin-facing surface and an opposing outwardly-facing surface, wherein the layer of anisotropic material (70) has a first thermal conductivity in a direction perpendicular to the skin-facing surface thereof and a thermal conductivity of the layer of anisotropic material (70) in directions parallel to the skin-facing surface thereof is more than two times the first thermal conductivity; and
a skin-contact layer (60; 61) comprising a conductive adhesive composite;
wherein the at least one electrode element (E1, E2) is in electrical contact with the outwardly-facing surface of the layer of anisotropic material (70), and the skin-contact layer (60; 61) is disposed on a skin-facing side of the layer of anisotropic material (70).

2. The apparatus of claim 1, wherein the conductive adhesive composite comprises a dielectric material and conductive particles dispersed within the dielectric material.

3. The apparatus of claim 2, wherein the conductive particles comprise graphite powder, carbon flakes, carbon granules, carbon fibers, carbon nanotubes, carbon nanowires or carbon black powder.

4. The apparatus of claim 1, wherein the layer of anisotropic material (70) is a synthetic graphite.

5. The apparatus of claim 1, wherein the layer of anisotropic material (70) is a sheet of pyrolytic graphite, a graphitized polymer film or a graphite foil of compressed high-purity exfoliated mineral graphite.

6. The apparatus of claim 1, wherein the skin-contact layer (60; 61) is disposed on the skin-facing surface of the layer of anisotropic material (70).

## Patentansprüche

1. Einrichtung, umfassend:
mindestens ein Elektrodenelement (E1, E2), das eine der Haut zugewandte Oberfläche aufweist;
eine Schicht aus anisotropem Material (70), die eine der Haut zugewandte Oberfläche und eine entgegengesetzte, nach außen gerichtete Oberfläche aufweist, wobei die Schicht aus anisotropem Material (70) eine erste Wärmeleitfähigkeit in einer Richtung, senkrecht zu der Haut zugewandten Oberfläche aufweist, und eine Wärmeleitfähigkeit der Schicht aus anisotropem Material (70) in Richtungen, parallel zu der Haut zugewandten Oberfläche, mehr als das Zweifache der ersten Wärmeleitfähigkeit beträgt; und
eine Hautkontaktschicht (60; 61), die einen leitfähigen Klebstoffverbundwerkstoff umfasst;
wobei das mindestens eine Elektrodenelement (E1, E2) in elektrischem Kontakt mit der nach außen gerichteten aus Oberfläche der Schicht anisotropem Material (70) steht und die Hautkontaktschicht (60; 61) auf der der Haut zugewandten Seite der Schicht aus anisotropem Material (70) angeordnet ist.

2. Einrichtung nach Anspruch 1, wobei der leitfähige Klebstoffverbund ein dielektrisches Material und innerhalb des dielektrischen Materials dispergierte leitfähige Partikel umfasst.

3. Einrichtung nach Anspruch 2, wobei die leitfähigen Partikel Graphitpulver, Kohlenstoffflocken, Kohlenstoffgranulat, Kohlenstofffasern, Kohlenstoffnanoröhren, Kohlenstoffnanodrähte oder Rußpulver umfassen.

4. Einrichtung nach Anspruch 1, wobei die Schicht aus anisotropem Material (70) ein synthetischer Graphit ist.

5. Einrichtung nach Anspruch 1, wobei die Schicht aus anisotropem Material (70) eine Schicht aus pyrolytischem Graphit, eine graphitierte Polymerfolie oder eine Graphitfolie aus komprimiertem, hochreinem, exfoliertem Mineralgraphit handelt.

6. Einrichtung nach Anspruch 1, wobei die Hautkontaktschicht (60; 61) auf der der Haut zugewandten Oberfläche der Schicht aus anisotropem Material (70) angeordnet ist.

## Revendications

1. Appareil, comprenant :
au moins un élément d'électrode (E1, E2) présentant une surface orientée vers la peau ;
une couche de matériau anisotrope (70) présentant une surface orientée vers la peau et une surface opposée orientée vers l'extérieur, dans lequel la couche de matériau anisotrope (70) présente une première conductivité thermique dans une direction perpendiculaire à sa surface orientée vers la peau, et une conductivité thermique de la couche de matériau anisotrope (70) dans les directions parallèles à sa surface orientée vers la peau est supérieure de plus de deux fois à la première conductivité thermique ; et
une couche en contact avec la peau (60 ; 61) comprenant un composite adhésif conducteur ;
dans lequel le au moins un élément d'électrode (E1, E2) est en contact électrique avec la surface orientée vers l'extérieur de la couche de matériau anisotrope (70), et la couche en contact avec la peau (60 ; 61) est disposée sur un côté de la couche de matériau anisotrope (70) orienté vers la peau.

2. Appareil selon la revendication 1, dans lequel le composite adhésif conducteur comprend un matériau diélectrique et des particules conductrices dispersées dans le matériau diélectrique.

3. Appareil selon la revendication 2, dans lequel les particules conductrices comprennent de la poudre de graphite, des flocons de carbone, des granules de carbone, des fibres de carbone, des nanotubes de carbone, des nanofils de carbone ou de la poudre de noir de carbone.

4. Appareil selon la revendication 1, dans lequel la couche de matériau anisotrope (70) est un graphite synthétique.

5. Appareil selon la revendication 1, dans lequel la couche de matériau anisotrope (70) est une feuille de graphite pyrolytique, un film polymère graphitisé ou une feuille de graphite minéral exfolié compressé de haute pureté.

6. Appareil selon la revendication 1, dans lequel la couche en contact avec la peau (60; 61) est disposée sur la surface orientée vers la peau de la couche de matériau anisotrope (70).
